# EUROPEAN PATENT APPLICATION

(11) **EP 4 803 194 A1**
(43) Date of publication of application: **09.09.2026**
(21) Application number: 24884943.2
(22) Date of filing: 31.10.2024
(51) Int. Cl.: B01J 29/14, B01J 29/76, B01J 29/46, C07C 2/62, C07C 2/86

(54) **N-ALKYLATION CATALYST, PREPARATION METHOD THEREFOR AND USE THEREOF**

(30) Priority: 01.11.2023 CN 202311442531; 05.07.2024 CN 202410900206
(71) Applicant: China Petroleum & Chemical Corporation, Beijing 100728 (CN); Sinopec Research Institute of Petroleum Processing Co., Ltd., Beijing 100083 (CN)
(72) Inventor: ZHOU, Shunli, Beijing 100083 (CN); LI, Yongxiang, Beijing 100083 (CN); ZHANG, Chengxi, Beijing 100083 (CN); FU, Qiang, Beijing 100083 (CN); XING, Enhui, Beijing 100083 (CN); SHU, Xingtian, Beijing 100083 (CN)
(74) Representative: Hoffmann Eitle
(86) International application number: PCT/CN2024/129145
(87) International publication number: WO 2025/092945

(57) **Abstract**

The present disclosure relates to an N-alkylation catalyst and a preparation method and use thereof. The catalyst comprises a molecular sieve, an oxide binder, and a modifying component. The modifying component is supported on both the molecular sieve and the oxide binder. The modifying component comprises Cu and P. The percentage of the medium-strong acid amount with respect to the total acid amount of the catalyst is not less than 35%. By controlling the addition order of raw materials during the preparation of the catalyst and selecting Cu and P for synergistic modification, an N-alkylation catalyst with high activity, high stability, extended lifetime, and good recycling performance can be obtained. When the catalyst is used for producing N,N-dimethylaniline, it enables to reduce side reactions, to achieve long-term stable operation of the N-alkylation reaction, and to broaden the applicable reaction temperature, which is beneficial for continuous industrial production.

## Description

### TECHNICAL FIELD

The present disclosure relates to an N-alkylation catalyst, a preparation method thereof, and use thereof in the synthesis of N,N-dimethylaniline (DMA).

### BACKGROUND ART

N,N-dimethylaniline, as an important chemical raw material or intermediate, is widely used in many fields such as papermaking, textile dyes, pharmaceuticals, spices, and explosives. For example, it can be used as a dye intermediate, solvent, stabilizer, and analytical reagent, etc. In the pharmaceutical industry, it can be used to manufacture drugs such as cephalosporin V, sulfamonomethoxine, sulfadoxine, and flucytosine. In the spice industry, it can be used to manufacture spices such as vanillin. In addition, it can also be used as an epoxy resin curing agent, a curing accelerator for polyester resins, a co-catalyst for polymerization of vinyl compounds, etc. With the development and innovation of the chemical industry, the application fields of N,N-dimethylaniline are still expanding, and the market demand is also showing a good growth trend.

DMA is mainly synthesized through the N-alkylation reaction of aniline. At present, the main methods for the N-alkylation reaction of aniline include a liquid phase method generally using sulfuric acid as a catalyst and a gas phase method generally using a solid acid as a catalyst. The sulfuric acid-catalyzed liquid phase method is currently the mainstream technology for industrial production of DMA. DMA production is conducted by the reaction of aniline and methanol in an autoclave under high temperature and high pressure conditions in a batch operation mode. This method has the advantages of high conversion rate and mature process, but suffers from the problems such as poor continuous production capacity, large equipment investment (because the reaction is conducted in the presence of a liquid acid catalyst and under high pressure, corrosion-resistant and high-pressure-resistant equipment materials are required), serious environmental pollution (for example, a large amount of acid-soluble oil is generated during the production process, and a large amount of liquid alkali is required to neutralize the inorganic acid in the separation of the product, resulting in a large amount of difficult-to-treat inorganic salts), and high post-treatment cost. Therefore, the development of an efficient and environmentally friendly green synthesis process for DMA has become a current research hotspot.

In the gas phase method, metal oxides, metal salts, molecular sieves, etc. are used as catalysts to synthesize DMA through the gas phase alkylation reaction of aniline and methanol. Although the gas phase method has been studied in places such as India, the product selectivity is relatively low (about 90%, lower than the liquid phase method), which limits its industrial application. However, the gas phase method has the potential for continuous production capacity and low waste generation, providing a new idea for the green synthesis of DMA. In recent years, with technological progress and improvement of catalysts, the selectivity and yield of the gas phase method have been improved, gradually becoming an important method for preparing DMA.

In China, a large number of studies have also been conducted on the gas phase method for synthesizing N,N-dimethylaniline. Wu Kerui et al. (Petrochemical Technology, 1988, 17(3): 135-137) reported, the selectivity for DMA was 88% by subjecting aniline and methanol to a reaction using modified ZSM-5 as a catalyst under the conditions of reaction pressure at atmospheric pressure, reaction temperature at 300°C, liquid hourly space velocity of 1.0 h⁻¹, and aniline to methanol molar ratio of 1:3. Li Guotao et al. (Petrochemical Technology, 2002, 31(2): 81-83) reported, the raw material conversion rate was about 99%, and the product selectivity was 85%, by using β molecular sieve as a catalyst, under the conditions of aniline to methanol molar ratio of 1:3, reaction temperature of 240-250°C, and space velocity of 0.5 h⁻¹. In the above literature, the reactant conversion rate and product selectivity are still relatively low, and the conversion rate and selectivity decrease rapidly as the reaction proceeds. How to improve the selectivity and stability of the catalyst is a problem to be solved for molecular sieve catalysts used in the synthesis of DMA through N-alkylation.

From the perspective of the reaction mechanism, the alkylation reaction of methanol and aniline to synthesize N,N-dimethylaniline is usually carried out at a high temperature of 300°C, and water molecules are generated during the reaction. Molecular sieves are prone to dealumination under high temperature and water-containing conditions, leading to permanent deactivation, which greatly affects the conversion rate, selectivity, and stability of the catalyst. In addition, the coking on the catalyst starts from the generation of by-product N,N,C-trimethylaniline from the alkylation of N,N-dimethylaniline and methanol, and then continues to undergo hydrogen transfer, alkylation, cyclization and other reactions to form non-volatile, polycyclic-structured coke deposits. These coke deposits are called hard coke, which is generally difficult to be removed unless high-temperature oxidation calcination is used. But high-temperature oxidation calcination will destroy the structure of the catalyst, reduce the acid amount of the catalyst, and thus reduce the activity and stability of the catalyst.

Researchers have tried to improve the activity and stability of catalysts by methods such as phosphorus modification. Generally speaking, existing phosphorus modification methods mostly focus on molecular sieve modification. A few documents report phosphorus modification of the catalyst after molding, but this phosphorus modification method for catalysts is relatively complicated and requires phosphorus modification and recalcination after the catalyst is calcined. Multiple calcinations not only have a great impact on the activity of the catalyst, but also increase the complexity of the catalyst preparation process and high energy consumption. Therefore, there is a need to develop a catalyst with high efficiency, stability, simple preparation process, and low energy consumption.

The gas phase method for synthesizing N,N-dimethylaniline still faces many challenges, including the improvement of catalytic performance and stability of the catalyst, as well as the economy and environmental friendliness of the process. By developing new efficient and stable catalysts, exploring simpler and controllable catalyst modification methods, and optimizing reaction conditions, constructing a continuous, low-energy-consumption production process, it is expected to realize the sustainable development of N,N-dimethylaniline production and provide strong support for the transformation and upgrading of related industries.

### SUMMARY OF THE INVENTION

The technical problem to be solved by the present disclosure is the problems existing in the prior art of low catalytic efficiency, poor catalyst stability, high cost, environmental pollution, etc. in the gas phase method for synthesizing N,N-dimethylaniline. To solve the above technical problems, the present disclosure provides a novel N-alkylation catalyst and a preparation method and use thereof. The N-alkylation catalyst is simple to prepare. When used in the gas phase method for synthesizing N,N-dimethylaniline, it exhibits the advantages of good stability, high reactant conversion rate, high target product selectivity, wide applicable reaction temperature range, and easy separation from the product, which is beneficial for realizing green and efficient production of N,N-dimethylaniline.

In a first aspect, the present disclosure provides an N-alkylation catalyst, characterized in that the catalyst comprises a molecular sieve, an oxide binder, and a modifying component, the modifying component is supported on both the molecular sieve and the oxide binder, the modifying component comprises Cu and P, the percentage of the medium-strong acid amount with respect to the total acid amount of the catalyst is not less than 35%, preferably not less than 40%, more preferably not less than 42%, the percentage of the medium-strong acid amount in the total acid amount is calculated as the percentage of the desorption peak area in a range of 250-450°C with respect to the desorption peak area of the total acid amount measured by NH₃ temperature-programmed desorption method.

In some embodiments, the molecular sieve is selected from at least one of Y-type molecular sieve, ZSM-5 type molecular sieve, β-type molecular sieve, MCM-22 type molecular sieve, and mordenite type molecular sieve, preferably at least one of Y-type molecular sieve, ZSM-5 type molecular sieve, and β-type molecular sieve, more preferably β-type molecular sieve. In some embodiments, the oxide binder is alumina and/or silica. In some embodiments, the Cu:P molar ratio calculated as oxides *n*(CuO): *n*(P₂O₅) in the catalyst is in a range of 30:1 to 1:25, preferably in a range of 20:1 to 1:10. In some embodiments, the mass ratio of the molecular sieve to the oxide binder is in a range of 95:5 to 20:80, preferably in a range of 90:10 to 25:75. In some embodiments, the ratio of Cu⁺/Cu²⁺ in the catalyst is in a range of 0-0.4, preferably in a range of 0.1-0.35. In some embodiments, the percentage of the pyridine infrared L acid amount with respect to the pyridine infrared total acid amount of the catalyst is not less than 60%, preferably in a range of 65-80%. In some embodiments, the modifying component further comprises a metal M component selected from at least one of Group VIII metals and Group VIB metals; preferably, the metal M component is selected from at least one of Cr, Zn, Fe, and Ni, more preferably selected from at least one of Cr, Zn, and Ni.

In a second aspect, the present disclosure provides a method for preparing the above N-alkylation catalyst, comprising:
1) mixing a molecular sieve and an oxide binder to obtain a mixture;
2) mixing the mixture obtained in step 1) with a solution containing a copper precursor and a phosphorus-containing compound, followed by molding, and then subjecting to a single calcination, to obtain a catalyst.

In some embodiments, in step 1), the oxide binder is a solid-state oxide binder or a sol-state oxide binder, preferably a sol-state oxide binder. In some embodiments, in step 1), the oxide binder is alumina and/or silica. In some embodiments, in step 1), the molecular sieve is selected from at least one of Y-type molecular sieve, ZSM-5 type molecular sieve, β-type molecular sieve, MCM-22 type molecular sieve, and mordenite, preferably at least one of Y-type molecular sieve, ZSM-5 type molecular sieve, and β-type molecular sieve, more preferably β-type molecular sieve. In some embodiments, in step 2), the copper precursor is selected from soluble copper compounds, preferably selected from at least one of copper chloride, cuprous chloride, copper nitrate, copper sulfate, and cuprous sulfate. In some embodiments, in step 2), the phosphorus-containing compound is selected from at least one of phosphoric acid, phosphorous acid, soluble phosphates, and phosphites; preferably, the soluble phosphate is selected from at least one of ammonium phosphate, diammonium hydrogen phosphate, and ammonium dihydrogen phosphate. In some embodiments, in step 2), calculated as elements, the mass ratio of the copper precursor to the phosphorus-containing compound is in a range of 5:95-98:2, preferably in a range of 30:70-80:20; preferably, the mass ratio of the oxide binder to the copper precursor calculated as elements is in a range of 80:20-99.9:0.1, preferably in a range of 85:15-99:1. In some embodiments, in step (2), the calcination is conducted under conditions including: temperature in a range of 450-800°C, preferably in a range of 500-700°C; time in a range of 1-15 h, preferably in a range of 1-12 h.

In some embodiments, when a sol-state oxide binder is used in step 1), the method further comprises: introducing a peptizing agent into the solution containing the copper precursor and the phosphorus-containing compound, mixing with the mixture obtained in step 1), followed by molding; preferably, the peptizing agent is selected from at least one of nitric acid, hydrochloric acid, sulfuric acid, phosphoric acid, formic acid, and acetic acid; preferably, the peptizing agent is used in such an amount that the pH of the solution containing the copper precursor and the phosphorus-containing compound is in a range of 1-4.

In some embodiments, the method further comprises: 3) subjecting the catalyst obtained in step 2) to an activation treatment to obtain an activated catalyst; preferably, the activation treatment is conducted under an activation atmosphere, preferably the activation atmosphere is selected from at least one of hydrogen, CO, ammonia, and hydrogen sulfide, more preferably hydrogen; preferably, the activation treatment is conducted under conditions including: temperature in a range of 150-600°C, preferably in a range of 200-400°C; time in a range of 0.5-30 h, preferably in a range of 2-24 h; flow rate of the activation atmosphere in a range of 0.001-100 mL/min/g catalyst, preferably in a range of 0.01-50 mL/min/g catalyst.

In some embodiments, step (2) of the method further comprises: introducing a metal M component precursor to mix with the mixture obtained in step 1), the copper precursor, and the phosphorus-containing compound, preferably, calculated as elements, the mass ratio of the metal M component precursor to the copper precursor is in a range of 1:50-1:1, preferably in a range of 1:25-1:2; preferably, the metal M component precursor is selected from soluble compounds of at least one of Group VIII metals and Group VIB metals, preferably selected from soluble compounds of at least one of Cr, Zn, Fe, and Ni, more preferably selected from at least one of chromium chloride, chromium nitrate, zinc nitrate, zinc sulfate, nickel nitrate, basic nickel carbonate, nickel acetate, nickel sulfate, iron nitrate, ferrous chloride, and ferric chloride.

In a third aspect, the present disclosure further provides an N-alkylation catalyst obtained by the preparation method according to the second aspect.

In a fourth aspect, the present disclosure further provides use of the N-alkylation catalyst according to the first aspect or the N-alkylation catalyst obtained by the preparation method according to the second aspect in an N-alkylation reaction of aniline, particularly in the production of N,N-dimethylaniline through an N-alkylation reaction of aniline.

In a fifth aspect, the present disclosure provides a method for producing N,N-dimethylaniline, comprising subjecting methanol and aniline to a reaction in the presence of a catalyst to produce N,N-dimethylaniline, wherein the reaction is a gas phase reaction; wherein, the catalyst is the N-alkylation catalyst according to the first aspect or the N-alkylation catalyst obtained by the preparation method according to the second aspect.

In some embodiments, the reaction is conducted under conditions including: molar ratio of methanol to aniline in a range of 1-10:1, preferably in a range of 2-8:1; reaction temperature in a range of 180-350°C, preferably in a range of 220-300°C; weight hourly space velocity of methanol and aniline in a range of 0.1-10 h⁻¹, preferably in a range of 0.5-4.5 h⁻¹.

The N-alkylation catalyst provided by the present disclosure, through the introduction of Cu and P for synergistic modification, on the one hand can adjust the acid amount distribution in the catalyst, increase the proportion of medium-strong acid amount in the catalyst, reduce the proportion of strong acid amount in the catalyst, and reduce the adsorption of methanol, which has relatively high polarity, on acid sites, thereby reducing the occurrence of side reactions, improving the catalytic performance of the catalyst, such as reactant conversion rate and target product selectivity; on the other hand, can improve the hydrothermal stability and/or recycling performance of the catalyst, broaden the applicable reaction temperature range for the N-alkylation reaction, realize long-period operation of the N,N-dimethylaniline production process, facilitate continuous industrial production, and greatly save production costs.

The N-alkylation catalyst provided by the present disclosure, through further adjusting the ratio of Cu⁺/Cu²⁺, significantly improves the activity, stability, and lifetime of the catalyst. In addition, the variable-valent metal selected as the metal M component itself has multiple valence states, which can also reduce the residence of the target product at acid sites, further improving the lifetime of the catalyst.

The preparation method of the N-alkylation catalyst provided by the present disclosure, by controlling the addition order of raw materials in the catalyst preparation process, strictly follows that the molecular sieve and the oxide binder are mixed, then directly mixed with the solution containing the copper precursor and the phosphorus-containing compound and other components without calcination, then molded, and subjected to a single calcination and preferably an activation treatment to obtain the catalyst. Only a single calcination is required in the catalyst preparation process, which avoids multiple calcinations of the catalyst modification after molding in the prior art, reduces the loss of acid amount caused by multiple calcinations, reduces the difficulty of the catalyst preparation process, and reduces energy consumption. Furthermore, through the activation treatment, the catalyst has a specific range of the ratio of Cu⁺/Cu²⁺, thereby significantly improving the activity, stability, and lifetime of the catalyst.

The method for producing N,N-dimethylaniline provided by the present disclosure, by adopting the catalyst with high activity, high stability, extended lifetime, and good recycling performance, can reduce the occurrence of side reactions, enable long-term stable operation of the N-alkylation reaction, and broaden the applicable reaction temperature, which is beneficial for continuous industrial production. The method also has the advantages of being green and environmentally friendly, recyclable and reusable catalyst, as well as zero pollution and zero emissions during the production process.

Other features and advantages of the present application will be described in detail in the subsequent detailed description.

### DETAILED DESCRIPTION

The specific embodiments of the present application are described in detail below. It should be understood that the specific embodiments described herein are only used to illustrate and explain the present application and are not intended to limit the present application.

Any specific numerical value (including the endpoints of numerical ranges) disclosed herein is not limited to the exact numerical value, but should be understood to also cover all possible numerical values close to the exact numerical value, such as all possible numerical values within a range of ±5% from the exact numerical value. Moreover, for the disclosed numerical ranges, one or more new numerical ranges can be obtained by arbitrary combination between the endpoint values of the range, between endpoint values and specific point values within the range, and between various specific point values. These new numerical ranges should also be considered as specifically disclosed herein.

Unless otherwise stated, the terms used herein have the same meanings as commonly understood by those skilled in the art. If a term is defined herein and its definition is different from the common understanding in the art, the definition herein shall prevail.

### N-alkylation catalyst

As described above, in a first aspect, the present disclosure provides an N-alkylation catalyst, characterized in that the catalyst comprises a molecular sieve, an oxide binder, and a modifying component, the modifying component is supported on both the molecular sieve and the oxide binder, the modifying component comprises Cu and P, the percentage of the medium-strong acid amount with respect to the total acid amount of the catalyst is not less than 35%, preferably not less than 40%, more preferably not less than 42%.

In the present disclosure, the acid amount distribution of the catalyst is characterized by NH₃ temperature-programmed desorption method (NH₃-TPD). The characterization method is as follows: Instrument: Quantachrome Chemstar TPx; Test process: Weigh 0.15 g (molded and ground to 20-40 mesh) of molecular sieve or catalyst sample, heat to 550°C for drying, then cool to 100°C to make the catalyst saturatedly adsorb NH₃, respectively heat to 250°C, 350°C, 450°C, and 550°C to desorb NH₃, and detect NH₃ concentration with a TCD detector. Integrate the adsorption curves obtained at different temperature segments, and the instrument automatically calculates the acid density distribution at different temperatures. Among them, the acid amount integrated in a range of 150-250°C is weak acid, 250-450°C is medium-strong acid, and 450-550°C is strong acid; 150-550°C is the total acid amount.

In some embodiments, the percentage of the medium-strong acid amount with respect to the total acid amount of the catalyst may be in a range of 35%-50%, preferably in a range of 40%-50%, more preferably in a range of 42%-50%. For example, the percentage of the medium-strong acid amount with respect to the total acid amount of the catalyst may be 35%, 36%, 37%, 38%, 39%, 40%, 41%, 42%, 43%, 44%, 45%, 46%, 47%, 48%, 49%, 50%, or even higher, or may be within a range between any two of the above values. The catalyst according to the present disclosure has a relatively high proportion of medium-strong acid amount. The medium-strong acid sites of the catalyst can reduce the adsorption of methanol, which has relatively high polarity, on the acid sites, reducing the occurrence of side reactions. On the other hand, the increased proportion of medium-strong acid amount of the catalyst allows to maintain a high conversion rate at a lowered reaction temperature, thereby further inhibiting the occurrence of side reactions at high temperatures, reducing coke formation, and extending catalyst lifetime.

In some embodiments, the percentage of the strong acid amount with respect to the total acid amount of the catalyst may be in a range of 5%-9%, preferably in a range of 5%-8.8%. For example, the percentage of the strong acid amount with respect to the total acid amount of the catalyst may be 9%, 8.5%, 8%, 7.5%, 7%, 6.5%, 6%, 5.5%, 5%, or even lower, or may be within a range between any two of the above values. The catalyst according to the present disclosure has a relatively low proportion of strong acid amount. The strong acid sites of the catalyst will promote the target product to further undergo undesired side reactions. Therefore, the reduced proportion of strong acid amount of the catalyst allows to reduce the occurrence of side reactions and improve the selectivity and stability of the catalyst.

In some embodiments, the molecular sieve is selected from at least one of Y-type molecular sieve, ZSM-5 type molecular sieve, β-type molecular sieve, MCM-22 type molecular sieve, and mordenite type molecular sieve, preferably at least one of Y-type molecular sieve, ZSM-5 type molecular sieve, and β-type molecular sieve. In a particularly preferred embodiment, the molecular sieve in the catalyst according to the present disclosure is a β-type molecular sieve. Such a catalyst exhibits high reactant conversion rate and high target product selectivity, and has an extended catalyst lifetime, when used in producing N,N-dimethylaniline through an N-alkylation reaction.

In some embodiments, the oxide binder is alumina and/or silica. The present disclosure, by simultaneously modifying both the molecular sieve and the oxide binder, further improves the total acid amount and the proportion of medium-strong acid amount of the catalyst, which is particularly advantageous for achieving the objectives of the present disclosure (e.g., improving the stability, catalytic performance, and service life of the catalyst).

In some embodiments, the mass ratio of the molecular sieve to the oxide binder is in a range of 95:5-20:80, preferably in a range of 90:10-25:75. For example, the mass ratio of the molecular sieve to the oxide binder may be 95:5, 90:10, 85:15, 80:20, 75:25, 70:30, 65:35, 60:40, 55:45, 50:50, 45:55, 40:60, 35:65, 30:70, 25:75, 20:80, or may be within a range between any two of the above values.

In some embodiments, the Cu molar content calculated as CuO in the catalyst is in a range of 0.01-10%, preferably in a range of 0.1-5%. For example, the Cu molar content calculated as CuO in the catalyst may be 0.01%, 0.05%, 0.1%, 0.2%, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, or may be within a range between any two of the above values.

In some embodiments, the P molar content calculated as P₂O₅ in the catalyst is in a range of 0.01-20%, preferably in a range of 0.05-15%. For example, the P molar content calculated as P₂O₅ in the catalyst may be 0.01%, 0.05%, 0.1%, 0.5%, 1%, 1.5%, 2%, 2.5%, 3%, 3.5%, 4%, 4.5%, 5%, 5.5%, 6%, 6.5%, 7%, 7.5%, 8%, 8.5%, 9%, 9.5%, 10%, 10.5%, 11%, 11.5%, 12%, 12.5%, 13%, 13.5%, 14%, 14.5%, 15%, 15.5%, 16%, 16.5%, 17%, 17.5%, 18%, 18.5%, 19%, 19.5%, 20%, or may be within a range between any two of the above values.

In some embodiments, the Cu:P molar ratio calculated as oxides *n*(CuO): *n*(P₂O₅) in the catalyst is in a range of 30:1-1:25, preferably in a range of 20:1-1:10. For example, the Cu:P molar ratio calculated as oxides *n*(CuO): *n*(P₂O₅) in the catalyst may be 30:1, 25:1, 20:1, 15:1, 10:1, 5:1, 2:1, 1:1, 1:2, 1:5, 1:10, 1:15, 1:20, 1:25, or may be within a range between any two of the above values.

By controlling the Cu content, P content, and/or Cu:P molar ratio in the catalyst, it is possible to adjust the acid amount distribution in the catalyst, and to increase the proportion of medium-strong acid amount, thereby improving the activity, hydrothermal stability, and/or recycling performance of the catalyst.

In some preferred embodiments, the ratio of Cu⁺/Cu²⁺ in the catalyst is in a range of 0-0.4, preferably in a range of 0.1-0.35. For example, the ratio of Cu⁺/Cu²⁺ in the catalyst may be 0, 0.05, 0.1, 0.15, 0.2, 0.25, 0.3, 0.35, 0.4, or may be within a range between any two of the above values. The catalyst according to the present disclosure preferably has a specific ratio of Cu⁺/Cu²⁺. The advantage of adopting such preferred embodiments is that the ratio of Cu⁺/Cu²⁺ being within a specific range allows to effectively reduce the adsorption capacity of aniline on acid sites, reduce the generation of coke precursors, and improve catalyst stability. In the prior art, the ratio of Cu⁺/Cu²⁺ is usually greater than 1.5. When the ratio of Cu⁺/Cu²⁺ is not within the above range, it is unable to reduce the adsorption capacity of aniline on acid sites, which is not conducive to improving the stability of the catalyst.

It should be noted that, in the present disclosure, the ratio of Cu⁺/Cu²⁺ refers to the ratio of the content of Cu⁺ to the content of Cu²⁺ in the whole catalyst.

In the present disclosure, the ratio of Cu⁺/Cu²⁺ is measured using the chemisorption module of a Micromeritics ASAP 2460 adsorption instrument (USA), with specific test conditions as follows:
(a) Catalyst pretreatment: Take 0.2 g of catalyst sample and vacuum pretreat at 330°C for 10 h to remove impurities on the catalyst surface;
(b) Carbon monoxide adsorption: Use carbon monoxide gas as the adsorption medium; at 25°C, in the relative pressure range of p/p0 = 0.05-0.65, the sample after impurity removal in step (a) is subjected to carbon monoxide adsorption to measure the total adsorption isotherm curve, and then the total adsorption area is calculated according to the BET equation, and the total adsorption area is counted as the total content A of Cu⁺ and Cu²⁺;
(c) Carbon monoxide desorption: Evacuate to 10⁻⁶ Pa at 25°C. Under these conditions, the sample after adsorption in step (b) is subjected to carbon monoxide desorption to measure the desorption isotherm curve (reversible adsorption isotherm curve), and then the desorption area (reversible adsorption area) is calculated according to the BET equation. At 25°C, the carbon monoxide adsorbed by Cu⁺ will not desorb, and the desorption area (reversible adsorption area) is counted as the content A1 of Cu²⁺;
(d) Calculate the irreversible adsorption area A2 = A - A1, count the irreversible adsorption area as the content A2 of Cu⁺, and then calculate the ratio A2/A1, Cu⁺/Cu²⁺ = A2/A1.

In some embodiments, a variable-valent metal selected from at least one of Group VIII metals and Group VIB metals is suitable for use as the metal M component in the present disclosure. Preferably, the metal M component is selected from at least one of Cr, Zn, Fe, and Ni, more preferably selected from at least one of Cr, Zn, and Ni. Preferably, with respect to the total weight of the catalyst, the molar content of the metal M component calculated as oxide in the catalyst is in a range of 0.01-5%, preferably in a range of 0.05-2%. For example, in some embodiments, the molar content of the metal M component calculated as oxide in the catalyst may be 0.01%, 0.05%, 0.1%, 0.5%, 1%, 1.5%, 2%, 2.5%, 3%, 3.5%, 4%, 4.5%, 5%, or may be within a range between any two of the above values.

In the present disclosure, Cu and the metal M component in the catalyst cooperate with each other to increase the proportion of L acid amount, maintain the ratio of Cu⁺/Cu²⁺ within a specific range, reduce the adsorption of reaction raw materials with relatively high polarity on acid sites, reduce the occurrence of side reactions, and improve the lifetime of the catalyst. Preferably, the percentage of the pyridine infrared L acid amount with respect to the pyridine infrared total acid amount of the catalyst is not less than 60%, more preferably in a range of 65-80%. For example, in some embodiments, the percentage of the pyridine infrared L acid amount with respect to the pyridine infrared total acid amount of the catalyst may be 65%, 66%, 67%, 68%, 69%, 70%, 71%, 72%, 73%, 74%, 75%, 76%, 77%, 78%, 79%, 80%, or may be within a range between any two of the above values.

In the present disclosure, the pyridine infrared acid amount of the catalyst is measured using a Bruker Tensor II Fourier transform infrared spectrometer. The specific test process: Take about 25 mg of catalyst sample, press into a tablet at 20 MPa for 5 min, the diameter of the tablet is 1.4 cm, place it in the in-situ cell of the infrared spectrometer and seal, heat to 450°C at a rate of 10°C/min, vacuum to about 10⁻⁶ Pa for 2 h, cool to room temperature, statically and saturatedly adsorb pyridine, then vacuum, stepwise heat to 200°C and 350°C to desorb the adsorbed pyridine, measure the infrared spectra at the two temperatures and integrate. The absorption peak at 1540 cm⁻¹ is attributed to Brönsted acid sites (B acid), and the absorption peak at 1450 cm⁻¹ is attributed to Lewis acid sites (L acid). The B acid amount and L acid amount are calculated by the following formulas: ${C}_{B} = 1.88 {A}_{B} {R}^{2} / W$ ${C}_{L} = 1.42 {A}_{L} {R}^{2} / W$
C represents the concentration of B acid or L acid (mmol/g);
A represents the integrated area of the absorption peak of B acid or L acid;
R represents the radius of the sample tablet (cm);
W represents the mass of the sample tablet (mg).

In the present disclosure, the content of each component in the catalyst is measured by X-ray fluorescence spectrometry (XRF) combined with the calculation of the feeding amount. The instrument used for X-ray fluorescence spectrometry is a ZSX-2 X-ray fluorescence spectrometer manufactured by Rigaku Corporation (Japan). The experimental parameters are set as follows: using a Rhodium target, setting the excitation voltage to 40 kV, the excitation current to 40 mA to ensure the stability and accuracy of the excitation source; sample processing and test conditions: the sample is processed in powder form, formed into a tight pellet by pressing, and the applied external pressure is generally set in a range of 500-1000 KPa to ensure that the sample surface is flat and uniform, facilitating effective penetration of X-rays and accurate collection of fluorescence signals; data analysis process: first, measure a standard sample, and construct a detailed working curve based on the measurement results. Then, by interpolation, locate the fluorescence intensity of the unknown sample on the working curve, thereby calculating the content of each component in the sample.

In the present disclosure, when component content is involved, unless otherwise stated, it is calculated as oxide, for example, Cu as CuO, P as P₂O₅, Cr as Cr₂O₃, Zn as ZnO, Fe as Fe₂O₃, Ni as NiO.

In the present disclosure, the catalyst contains micropores and mesopores. It should be noted that the mesopores of the catalyst refer to pores with a pore diameter in a range of 2-50 nm, and the micropores refer to pores with a pore diameter below 2 nm.

In some embodiments, the percentage of the mesopore volume with respect to the total pore volume of the catalyst is 30-55%, more preferably 40-50%. In some embodiments, the micropore volume of the catalyst is in a range of 0.05-0.3 cm³/g, more preferably in a range of 0.1-0.2 cm³/g. In some embodiments, the mesopore volume of the catalyst is in a range of 0.05-0.3 cm³/g, more preferably in a range of 0.05-0.25 cm³/g. In some embodiments, the total pore volume of the catalyst is in a range of 0.15-0.6 cm³/g, more preferably in a range of 0.15-0.45 cm³/g.

In the present disclosure, the pore volume of the catalyst is measured by the BET method using a Micromeritics ASAP 2460 multi-station specific surface area and porosimetry analyzer. The specific test process: the catalyst sample is evacuated to 10⁻³ Pa at 330°C and kept at low temperature for 9 h to remove water and other impurities adsorbed in the catalyst. The specific surface area and micropore area of the sample are calculated by the BET (Brunauer-Emmett-Teller) method; the desorption data are calculated by the BJH (Barrett-Joyner-Halenda) method to obtain the pore structure parameters of the sample.

The N-alkylation catalyst provided by the present disclosure, through the introduction of Cu and P for synergistic modification, can adjust the acid amount distribution in the catalyst, improve the catalytic performance, hydrothermal stability and/or recycling performance of the catalyst; by adjusting the ratio of Cu⁺/Cu²⁺, further significantly improves the activity, stability and lifetime of the catalyst, thereby broadening the applicable reaction temperature range for the N-alkylation reaction, realizing long-period operation of the N,N-dimethylaniline production process, facilitating continuous industrial production, and greatly saving production costs.

### Preparation method of N-alkylation catalyst

In a second aspect, the present disclosure provides the preparation method of the above N-alkylation catalyst, comprising:
1) mixing a molecular sieve and an oxide binder to obtain a mixture;
2) mixing the mixture obtained in step 1) with a solution containing a copper precursor and a phosphorus-containing compound, followed by molding, and then subjecting to a single calcination, to obtain a catalyst.

In some embodiments, in step 1), the oxide binder is a solid-state oxide binder or a sol-state oxide binder, preferably a sol-state oxide binder. In a particularly preferred embodiment, in step 1), the oxide binder is a sol-state oxide binder, such as an alumina sol and/or a silica sol. The sol-state oxide binder is conducive to improving dispersibility, achieving better mixing and more uniform distribution of the molecular sieve and the oxide binder in the catalyst, strengthening the interaction, and facilitating the improvement of the stability and catalytic performance of the catalyst.

In the present disclosure, the concentration of the sol-state oxide binder is not particularly limited. Preferably, the concentration of the sol-state oxide binder may be in a range of 0.1-50 wt%, more preferably in a range of 10-30 wt%. Preferably, the pH of the sol-state oxide binder may be in a range of 1-5, for example, 1, 2, 3, 4, 5, or may be within a range between any two of the above values. Preferably, the average particle size of the sol-state oxide binder is in a range of 0.1-300 nm.

In some embodiments, in step 1), the oxide binder is alumina and/or silica. In the present disclosure, the types of alumina and silica precursors are not particularly limited. Substances that can be converted into alumina and/or silica by calcination as conventionally defined in the art are applicable to the present disclosure. In some embodiments, in step 1), the oxide binder is alumina. Preferably, the alumina is provided by at least one of pseudoboehmite, aluminum chloride, aluminum hydroxide, and an alumina sol. In some embodiments, when a sol-state oxide binder is used in step 1), the oxide binder is an alumina sol and/or a silica sol.

In some embodiments, in step 1), the molecular sieve is selected from at least one of Y-type molecular sieve, ZSM-5 type molecular sieve, β-type molecular sieve, MCM-22 type molecular sieve, and mordenite, preferably at least one of Y-type molecular sieve, ZSM-5 type molecular sieve, and β-type molecular sieve, more preferably β-type molecular sieve. In some embodiments, in step 1), the silica-alumina molar ratio *n*(SiO₂): *n*(Al₂O₃) of the molecular sieve is in a range of 2-1000:1, preferably in a range of 2-100:1. In some embodiments, the average particle size of the molecular sieve is in a range of 0.01-50 µm, preferably in a range of 0.1-20 µm.

In some embodiments, in step 1), the mass ratio of the molecular sieve to the oxide binder is in a range of 95:5-20:80, preferably in a range of 90:10-25:75. For example, in step 1), the mass ratio of the molecular sieve to the oxide binder may be 95:5, 90:10, 85:15, 80:20, 75:25, 70:30, 65:35, 60:40, 55:45, 50:50, 45:55, 40:60, 35:65, 30:70, 25:75, 20:80, or may be within a range between any two of the above values.

In the present disclosure, the type of the copper precursor is not particularly limited as long as it can provide the copper element. In some embodiments, in step 2), the copper precursor is selected from soluble copper compounds. Preferably, the copper precursor is selected from at least one of copper chloride, cuprous chloride, copper nitrate, copper sulfate, and cuprous sulfate.

In the present disclosure, the type of the phosphorus-containing compound is not particularly limited as long as it can provide the phosphorus element. In some embodiments, in step 2), the phosphorus-containing compound is selected from at least one of phosphoric acid, phosphorous acid, soluble phosphates and phosphites. Preferably, the soluble phosphate is selected from at least one of ammonium phosphate, diammonium hydrogen phosphate, and ammonium dihydrogen phosphate. In some embodiments, the phosphorus-containing compound is provided in the form of an aqueous solution of the phosphorus-containing compound. Preferably, the concentration of the aqueous solution of the phosphorus-containing compound is in a range of 0.01-25 wt%, preferably in a range of 5-25 wt%.

In some embodiments, in step 2), calculated as elements, the mass ratio of the copper precursor to the phosphorus-containing compound is in a range of 5:95-98:2, preferably in a range of 30:70-80:20. By controlling the amounts of the copper precursor and the phosphorus-containing compound, it is possible to adjust the acid amount distribution in the catalyst, and to increase the proportion of medium-strong acid amount, thereby improving the activity, hydrothermal stability, and/or recycling performance of the catalyst.

In some embodiments, the mass ratio of the oxide binder to the copper precursor calculated as elements is in a range of 80:20-99.9:0.1, preferably in a range of 85:15-99:1.

In some embodiments, when the oxide binder exists in a sol state, a peptizing agent needs to be introduced to peptize it. Preferably, when a sol-state oxide binder is used in step 1), the method further comprises: introducing a peptizing agent into the solution containing the copper precursor and the phosphorus-containing compound, mixing with the mixture obtained in step 1), and followed by molding.

In the present disclosure, the type of the peptizing agent is not particularly limited, and peptizing agents conventionally defined in the art are applicable to the present disclosure. In some embodiments, the peptizing agent is selected from at least one of nitric acid, hydrochloric acid, sulfuric acid, phosphoric acid, formic acid, and acetic acid. Preferably, the peptizing agent is used in such an amount that the pH of the solution containing the copper precursor and the phosphorus-containing compound is in a range of 1-4.

In the present disclosure, the molding method in step 2) is not particularly limited, and those skilled in the art can select an appropriate method according to practical needs, such as ball molding, tablet molding, strand-extrusion molding, etc. For example, in a specific embodiment, extrusion molding is performed using an extruder.

In some embodiments, in step 2), the calcination is conducted under conditions including: temperature in a range of 450-800°C, time in a range of 1-15 h; preferably, in step 2), the calcination is conducted under conditions including: temperature in a range of 500-700°C, time in a range of 1-12 h. The atmosphere of the calcination treatment is an air atmosphere or a water vapor atmosphere.

In some preferred embodiments, the method further comprises: 3) subjecting the catalyst obtained in step 2) to an activation treatment to obtain an activated catalyst. Preferably, the activation treatment is conducted under an activation atmosphere, preferably a reducing atmosphere. Preferably, the activation atmosphere is selected from at least one of hydrogen, CO, ammonia, and hydrogen sulfide, more preferably hydrogen. The advantage of adopting such preferred embodiments is that the purity of hydrogen is relatively high, the degree of activation is easier to control, and it is easier to implement in industrial applications.

Preferably, in step 3), the activation treatment is conducted under conditions including: temperature in a range of 150-600°C, preferably in a range of 200-400°C; time in a range of 0.5-30 h, preferably in a range of 2-24 h; flow rate of the activation atmosphere in a range of 0.001-100 mL/min/g catalyst, preferably in a range of 0.01-50 mL/min/g catalyst. The advantage of adopting such preferred embodiments is that the ratio of Cu⁺/Cu²⁺ in the catalyst can be adjusted by the activation treatment to a specific range (for example, 0.1-0.35), which is beneficial for further significantly improving the stability and lifetime of the catalyst.

In some preferred embodiments, step 2) further comprises: introducing a metal M component precursor to mix with the mixture obtained in step 1), the copper precursor, and the phosphorus-containing compound. Preferably, calculated as elements, the mass ratio of the metal M component precursor to the copper precursor is in a range of 1:50-1:1, preferably in a range of 1:25-1:2. Preferably, the metal M component precursor is selected from soluble compounds of at least one of Group VIII metals and Group VIB metals, preferably selected from soluble compounds of at least one of Cr, Zn, Fe, and Ni, more preferably selected from at least one of chromium chloride, chromium nitrate, zinc nitrate, zinc sulfate, nickel nitrate, basic nickel carbonate, nickel acetate, nickel sulfate, iron nitrate, ferrous chloride, and ferric chloride. The advantage of adopting such preferred embodiments is that the metal M component cooperates with other modifying components (e.g., Cu) to increase the proportion of L acid amount, maintain the ratio of Cu⁺/Cu²⁺ within a specific range, reduce the adsorption of reaction raw materials with relatively high polarity on acid sites, reduce the occurrence of side reactions, and improve the lifetime of the catalyst.

In the present disclosure, step 2) may further comprise: before calcination, subjecting the molded catalyst precursor to a hydrothermal treatment. Preferably, in step 2), the hydrothermal treatment is conducted under the conditions including: temperature in a range of 80-400°C, preferably in a range of 120-250°C; time in a range of 0.5-72 h, preferably in a range of 8-48 h. The advantage of adopting such preferred embodiments is that the conditions are relatively mild, which can ensure that the framework structure of the catalyst is not destroyed, so that the catalyst has an excellent pore size structure.

In the present disclosure, the "hydrothermal treatment" refers to a broad sense of hydrothermal reaction: in a closed system such as an autoclave, in the presence of an aqueous solvent or a non-aqueous solvent, the reaction of the raw mixture under a certain temperature and the autogenous pressure of the solution. It can be understood that, in the present disclosure, the hydrothermal reaction solution system depends on the type of the solvent and does not necessarily contain water. Preferably, the solvent is water. The source of the solvent is not particularly limited in the present disclosure, and it can be provided by an external solvent or by the solvent of the raw material precursor solution itself.

The preparation method of the N-alkylation catalyst provided by the present disclosure, by controlling the addition order of raw materials in the catalyst preparation process, strictly follows that the molecular sieve and the oxide binder are mixed, then directly mixed with the solution containing the copper precursor and the phosphorus-containing compound and other components without calcination, then molded, and subjected to a single calcination and preferably an activation treatment to obtain the catalyst. Only a single calcination is required in the catalyst preparation process, which avoids multiple calcinations of the catalyst modification after molding in the prior art, reduces the loss of acid amount caused by multiple calcinations, reduces the difficulty of the catalyst preparation process, and reduces energy consumption. Furthermore, through the activation treatment, the ratio of Cu⁺/Cu²⁺ in the catalyst is adjusted to a specific range, which is beneficial for further significantly improving the stability and lifetime of the catalyst.

In a third aspect, the present disclosure further provides an N-alkylation catalyst obtained by the preparation method according to the second aspect.

In a fourth aspect, the present disclosure further provides use of the N-alkylation catalyst according to the first aspect or the N-alkylation catalyst obtained by the preparation method according to the second aspect in an N-alkylation reaction of aniline, particularly in the production of N,N-dimethylaniline through an N-alkylation reaction of aniline.

### Method for producing N,N-dimethylaniline

In a fifth aspect, the present disclosure provides a method for producing N,N-dimethylaniline, comprising subjecting methanol and aniline to a reaction in the presence of a catalyst to produce N,N-dimethylaniline, wherein the reaction is a gas phase reaction; wherein, the catalyst is the N-alkylation catalyst according to the first aspect or the N-alkylation catalyst obtained by the preparation method according to the second aspect.

It should be noted that, in the present disclosure, the gas phase reaction means that the reaction raw materials, i.e., methanol and aniline, are fed in form of gas phase or the reaction conditions during the reaction render methanol and aniline in a gas phase state. The gasification method of the raw materials is not particularly limited in the present disclosure, and gasification methods conventionally defined in the art are applicable to the present disclosure.

In some embodiments, the molar ratio of methanol to aniline may be in a range of 1-10:1, preferably in a range of 2-8:1, for example, 1:1, 2:1, 3:1, 4:1, 5:1, 6:1, 7:1, 8:1, 9:1, 10:1, or may be within a range between any two of the above values.

By adopting the N-alkylation catalyst according to the present disclosure, the applicable reaction temperature range can be broadened, allowing a high conversion rate to be maintained even at a reduced reaction temperature (for example, as low as 230°C). In some embodiments, the reaction temperature may be in a range of 180-350°C, preferably in a range of 220-300°C, for example, 180°C, 190°C, 200°C, 210°C, 220°C, 230°C, 240°C, 250°C, 260°C, 270°C, 280°C, 290°C, 300°C, 310°C, 320°C, 330°C, 340°C, 350°C, or may be within a range between any two of the above values.

In some embodiments, the weight hourly space velocity of methanol and aniline is in a range of 0.1-10 h⁻¹, preferably in a range of 0.5-4.5 h⁻¹, for example, 0.1 h⁻¹, 0.5 h⁻¹, 1 h⁻¹, 2 h⁻¹, 3 h⁻¹, 4 h⁻¹, 5 h⁻¹, 6 h⁻¹, 7 h⁻¹, 8 h⁻¹, 9 h⁻¹, 10 h⁻¹, or may be within a range between any two of the above values.

In some embodiments, the method is conducted in at least one of a fixed bed reactor, a fluidized bed reactor, a moving bed reactor, and a slurry bed reactor, preferably in a fixed bed reactor.

The method for producing N,N-dimethylaniline provided by the present disclosure, by adopting the catalyst with high activity, high stability, extended lifetime, and good recycling performance, can reduce the occurrence of side reactions, enable long-term stable operation of the N-alkylation reaction, and broaden the applicable reaction temperature, which is beneficial for continuous industrial production. The method also has the advantages of being green and environmentally friendly, recyclable and reusable catalyst, as well as zero pollution and zero emissions during the production process.

The present application also provides the following embodiments:
Embodiment 1. A method for producing N,N-dimethylaniline, characterized in that, in the presence of a catalyst, the reaction raw materials methanol and aniline are subjected to a reaction, and the reaction is a gas phase reaction;
   wherein, the preparation method of the catalyst comprises:
   (1) mixing a molecular sieve and a support to obtain a mixture;
   (2) mixing the mixture obtained in step (1) with a solution containing a copper precursor and a phosphorus-containing compound, molding, and then subjecting to a single calcination, to obtain a catalyst, wherein the percentage of the medium-strong acid amount with respect to the total acid amount of the catalyst is not less than 35%.
Embodiment 2. The method according to embodiment 1, wherein the percentage of the medium-strong acid amount with respect to the total acid amount of the catalyst is in a range of 40-50%, preferably in a range of 42-45%.
Embodiment 3. The method according to embodiment 1 or 2, wherein in step (1), the molecular sieve is selected from at least one of Y-type molecular sieve, ZSM-5 type molecular sieve, β-type molecular sieve, MCM-22 type molecular sieve, and mordenite, preferably at least one of Y-type molecular sieve, ZSM-5 type molecular sieve, and β-type molecular sieve, more preferably β-type molecular sieve;
   preferably, in step (1), the support is alumina and/or silica;
   preferably, in step (1), the mass ratio of the molecular sieve to the support calculated as oxide is in a range of 95:5-20:80, more preferably in a range of 90:10-25:75.
Embodiment 4. The method according to embodiment 3, wherein in step (1), the support exists in a solid state and/or a sol state, more preferably exists in a sol state;
   preferably, in step (1), the support is alumina.
Embodiment 5. The method according to any one of embodiments 1-4, wherein in step (2), the copper precursor is selected from soluble copper compounds, preferably selected from at least one of copper chloride, cuprous chloride, copper nitrate, copper sulfate, and cuprous sulfate;
   preferably, in step (2), the phosphorus-containing compound is selected from at least one of phosphoric acid, phosphorous acid, soluble phosphates and phosphites;
   preferably, the soluble phosphate is selected from at least one of ammonium phosphate, diammonium hydrogen phosphate, and ammonium dihydrogen phosphate.
Embodiment 6. The method according to any one of embodiments 1-5, wherein in step (2), calculated as elements, the mass ratio of the copper precursor to the phosphorus-containing compound is in a range of 5:95-98:2, preferably in a range of 30:70-80:20;
   preferably, the mass ratio of the support calculated as oxide to the copper precursor calculated as elements is in a range of 80:20-99.9:0.1, more preferably in a range of 85:15-99:1.
Embodiment 7. The method according to embodiment 4, wherein the preparation method of the catalyst further comprises: introducing a peptizing agent into the solution containing the copper precursor and the phosphorus-containing compound, mixing with the mixture obtained in step (1), and molding;
   preferably, the peptizing agent is selected from at least one of nitric acid, hydrochloric acid, sulfuric acid, phosphoric acid, formic acid, and acetic acid;
   preferably, the peptizing agent is used in such an amount that the pH of the solution containing the copper precursor and the phosphorus-containing compound is in a range of 1-4.
Embodiment 8. The method according to any one of embodiments 1-7, wherein in step (2), the calcination is conducted under conditions including: temperature in a range of 450-800°C, time in a range of 1-15 h;
   preferably, in step (2), the calcination is conducted under conditions including: temperature in a range of 500-700°C, time in a range of 2-8 h.
Embodiment 9. The method according to any one of embodiments 1-8, wherein the molar ratio of methanol to aniline is in a range of 1-10:1, preferably in a range of 2-8:1.
Embodiment 10. The method according to any one of embodiments 1-9, wherein the reaction is conducted under conditions including: reaction temperature in a range of 220-300°C, weight hourly space velocity of methanol and aniline in a range of 0.1-10 h⁻¹;
   preferably, the reaction is conducted under conditions including: reaction temperature in a range of 230-290°C, weight hourly space velocity of methanol and aniline in a range of 0.2-8 h⁻¹.
Embodiment 11. An alkylation catalyst, wherein the catalyst comprises Cu, a co-active component, and a support; the co-active component is selected from at least one of Group VIII metals and Group VIB metals; the support comprises a molecular sieve and a binder; wherein the ratio of Cu⁺/Cu²⁺ in the catalyst is in a range of 0.1-0.4.
Embodiment 12. The catalyst according to embodiment 11, wherein the ratio of Cu⁺/Cu²⁺ in the catalyst is in a range of 0.2-0.35;
   and/or, the percentage of the pyridine infrared total L acid amount with respect to the pyridine infrared total acid amount of the catalyst is not less than 60%, preferably in a range of 65-80%;
   and/or, the catalyst contains mesopores and micropores;
   preferably, the percentage of the mesopore volume with respect to the total pore volume of the catalyst is in a range of 30-55%, more preferably in a range of 40-50%.
Embodiment 13. The catalyst according to embodiment 11 or 12, wherein the co-active component is selected from at least one of Cr, Zn, Fe, and Ni, preferably selected from at least one of Cr, Zn, and Ni;
   preferably, the molecular sieve is selected from at least one of Y-type molecular sieve, ZSM-5 molecular sieve, β-type molecular sieve, MCM-22 type molecular sieve, and mordenite, more preferably at least one of Y-type molecular sieve, ZSM-5 molecular sieve, and β-type molecular sieve, still more preferably β-type molecular sieve;
   preferably, the binder is alumina and/or silica.
Embodiment 14. The catalyst according to any one of embodiments 11-13, wherein, with respect to the total weight of the catalyst, in the catalyst, the content of Cu calculated as oxide is in a range of 0.5-20%, the content of the co-active component calculated as oxide is in a range of 0.1-10%, the content of the molecular sieve is in a range of 20-92%, and the content of the binder calculated as oxide is in a range of 6.9-78.9%;
   preferably, with respect to the total weight of the catalyst, in the catalyst, the content of Cu calculated as oxide is in a range of 1-18%, the content of the co-active component calculated as oxide is in a range of 0.2-1%, the content of the molecular sieve is in a range of 60-80%, and the content of the binder calculated as oxide is in a range of 7-35%.
Embodiment 15. The catalyst according to embodiment 14, wherein the catalyst further comprises an auxiliary agent;
   preferably, the auxiliary agent is selected from at least one of phosphorus, fluorine, and boron;
   preferably, with respect to the total weight of the catalyst, in the catalyst, the content of the auxiliary agent calculated as oxide is in a range of 0.5-12%, more preferably in a range of 0.8-9%.
Embodiment 16. A method for preparing an alkylation catalyst, wherein the preparation method comprises the following steps:
   (1) mixing a molecular sieve and a binder, followed by molding, to obtain a support precursor;
   (2) in the presence of a solvent, mixing the support precursor, a Cu precursor, and a co-active component precursor, and then performing a hydrothermal treatment to obtain the catalyst precursor;
   (3) subjecting the catalyst precursor to a calcination and then to an activation treatment;
   wherein the co-active component is selected from at least one of Group VIII metals and Group VIB metals.
Embodiment 17. The method according to embodiment 16, wherein in step (1), the silica-alumina molar ratio of the molecular sieve is in a range of 3-1000;
   preferably, in step (1), the molecular sieve is selected from at least one of Y-type molecular sieve, ZSM-5 molecular sieve, β-type molecular sieve, MCM-22 type molecular sieve, and mordenite, more preferably at least one of Y-type molecular sieve, ZSM-5 molecular sieve, and β-type molecular sieve, still more preferably β-type molecular sieve;
   and/or, in step (1), the binder is alumina and/or silica;
   preferably, in step (1), the binder is provided in a solid state and/or a sol state.
Embodiment 18. The method according to embodiment 16 or 17, wherein in step (2), the Cu precursor is selected from soluble compounds of Cu, preferably selected from at least one of copper chloride, cuprous chloride, copper nitrate, copper sulfate, and cuprous sulfate;
   preferably, in step (2), the co-active component is selected from at least one of Cr, Zn, Fe, and Ni, more preferably selected from at least one of Cr, Zn, and Ni;
   preferably, in step (2), the co-active component precursor is selected from soluble compounds of each co-active component, more preferably selected from at least one of chromium chloride, chromium nitrate, zinc nitrate, zinc sulfate, nickel nitrate, basic nickel carbonate, nickel acetate, nickel sulfate, iron nitrate, ferrous chloride, and ferric chloride;
   preferably, in step (2), the hydrothermal treatment is conducted under the conditions including: temperature in a range of 80-400°C, time in a range of 0.5-72 h;
   more preferably, in step (2), the hydrothermal treatment is conducted under the conditions including: temperature in a range of 120-250°C, time in a range of 8-48 h.
Embodiment 19. The method according to any one of embodiments 16-18, wherein the molecular sieve, the binder, the Cu precursor, and the co-active component precursor are used in such amounts that in the obtained catalyst, with respect to the total weight of the catalyst, the content of Cu calculated as oxide is in a range of 0.5-20%, the content of the co-active component calculated as oxide is in a range of 0.1-10%, the content of the molecular sieve is in a range of 20-92%, and the content of the binder calculated as oxide is in a range of 6.9-78.9%;
   preferably, the molecular sieve, the binder, the Cu precursor, and the co-active component precursor are used in such amounts that in the obtained catalyst, with respect to the total weight of the catalyst, the content of Cu calculated as oxide is in a range of 1-18%, the content of the co-active component calculated as oxide is in a range of 0.2-1%, the content of the molecular sieve is in a range of 60-80%, and the content of the binder calculated as oxide is in a range of 7-35%.
Embodiment 20. The method according to embodiment 19, wherein step (2) further comprises: introducing an auxiliary agent precursor to mix with the support precursor, the Cu precursor, and the co-active component precursor, and then performing a hydrothermal treatment to obtain the catalyst precursor;
   preferably, in step (2), the auxiliary agent is selected from at least one of phosphorus, fluorine, and boron;
   preferably, the auxiliary agent precursor is used in such an amount that in the obtained catalyst, with respect to the total weight of the catalyst, the content of the auxiliary agent calculated as oxide is in a range of 0.5-12%, more preferably in a range of 0.8-9%.
Embodiment 21. The method according to any one of embodiments 16-20, wherein in step (3), the calcination is conducted under conditions including: temperature in a range of 300-800°C, time in a range of 0.5-24 h;
   preferably, in step (3), the activation treatment is conducted under an activation atmosphere, preferably the activation atmosphere is selected from at least one of hydrogen, CO, ammonia, and hydrogen sulfide, more preferably hydrogen;
   preferably, in step (3), the activation treatment is conducted under conditions including: temperature in a range of 150-600°C, time in a range of 0.5-30 h, and with respect to 1 g of catalyst precursor, the flow rate of the activation atmosphere is in a range of 5-5000 mL/min;
   more preferably, in step (3), the activation treatment is conducted under conditions including: temperature in a range of 200-400°C, time in a range of 2-24 h, and with respect to 1 g of catalyst precursor, the flow rate of the activation atmosphere is in a range of 100-1200 mL/min.
Embodiment 22. An alkylation catalyst prepared by the preparation method according to any one of embodiments 16-21.
Embodiment 23. Use of the alkylation catalyst according to any one of embodiments 11-15 and 22 in an N-alkylation reaction of aniline.
Embodiment 24. A method for synthesizing N,N-dimethylaniline, wherein the synthesis method comprises: in the presence of a catalyst, the reaction raw materials aniline and methanol are subjected to an alkylation reaction, and the catalyst is the alkylation catalyst according to any one of embodiments 11-15 and 22.
Embodiment 25. The synthesis method according to embodiment 24, wherein the reaction is a gas phase reaction;
   preferably, the alkylation reaction is conducted under conditions including: reaction temperature in a range of 180-350°C, weight hourly space velocity of aniline and methanol in a range of 0.01-5 h⁻¹, and molar ratio of aniline to methanol in a range of 1:1-20;
   more preferably, the alkylation reaction is conducted under conditions including: reaction temperature in a range of 190-300°C, weight hourly space velocity of aniline and methanol in a range of 0.5-4.5 h⁻¹, and molar ratio of aniline to methanol in a range of 1: 1.5-18.

To facilitate understanding of the present disclosure, the present disclosure provides examples as follows. However, these examples are only for helping to understand the present disclosure and should not be considered as specific limitations on the present disclosure.

### EXAMPLES

The present disclosure will be described in detail below by means of examples. In the following examples, unless otherwise specified, all raw materials used are commercially available. Among them, the molecular sieve and oxide binder materials were purchased from Sinopec Catalyst Company.

In the following examples and comparative examples, the conversion rate of aniline and the selectivity distribution of the obtained products N,N-dimethylaniline and N-methylaniline were measured by gas chromatography (Agilent-7890) equipped with a high-pressure injector (chromatographic column: HP-PONA 50 m × 0.2 mm capillary column). The method for determining the single-pass lifetime of the catalyst was as follows: when the conversion rate of aniline decreased to 98%, the catalyst was considered to be deactivated, and the reaction time experienced by the catalyst was the single-pass lifetime of the catalyst.

In the following examples and comparative examples, the component contents of the catalyst, such as Al₂O₃, SiO₂, CuO, P₂O₅, Cr₂O₃, ZnO, Fe₂O₃, NiO content, etc., were measured by X-ray fluorescence spectrometry combined with the calculation of the feeding amount (see the detailed description).

In the following examples and comparative examples, the acid amount distribution of the catalyst was measured by the NH₃ temperature-programmed desorption method (NH₃-TPD) (see the detailed description).

In the following examples and comparative examples, the pyridine infrared acid amount of the catalyst was measured using a Bruker Tensor II Fourier transform infrared spectrometer (see the detailed description).

In the following examples and comparative examples, the ratio of Cu⁺/Cu²⁺ of the catalyst was measured using the chemisorption module of a Micromeritics ASAP 2460 adsorption instrument (see the detailed description).

### Example 1

1) 90 g of HY molecular sieve (silica-alumina molar ratio *n*(SiO₂): *n*(Al₂O₃) of 5, average particle size of 1 µm) and 10 g of alumina powder (average particle size of 20 µm) were mixed uniformly;
2) 3 g of cuprous chloride was added to 100 ml of a 10 wt% H₃PO₄ solution, then added to the mixture of step 1), mixed uniformly, extruded and molded using an extruder, and calcined at 500°C for 12 h to obtain catalyst Y-1. The component contents measured by X-ray fluorescence method were: SiO₂: 71.39%, Al₂O₃: 23.22%, CuO: 2.00%, P₂O₅: 3.39%. The acid amount results of catalyst Y-1 are shown in Table 1.

Methanol and aniline were reacted in a fixed bed reactor using catalyst Y-1, with a methanol to aniline molar ratio of 3:1, a weight hourly space velocity of methanol and aniline of 1 h⁻¹, and a reaction temperature of 250°C. The raw materials were pumped into the reactor by a raw material pump, and aniline and methanol were vaporized in the reactor and contacted with the catalyst in the catalyst bed to undergo the reaction. The reaction results are shown in Table 2.

### Example 2

1) 750 g of β molecular sieve (silica-alumina molar ratio *n*(SiO₂): *n*(Al₂O₃) of 20, average particle size of 1.5 µm) and 1250 ml of a 20 wt% alumina sol (average particle size of 10 nm) were mixed uniformly;
2) 5 g of Cu₂SO₄ was added to 100 ml of a 15 wt% KH₂PO₄ solution, then the pH was adjusted to 2.5 with nitric acid, and then added to the mixture of step 1), mixed uniformly, extruded and molded using an extruder, and calcined at 600°C for 1 h to obtain catalyst β-1. The component contents measured by X-ray fluorescence method were: SiO₂: 78.86%, Al₂O₃: 20.72%, CuO: 0.31%, P₂O₅: 0.38%. The acid amount results of catalyst β-1 are shown in Table 1.

The reaction was carried out using catalyst β-1 according to the method and reaction conditions of Example 1. The reaction results are shown in Table 2.

### Example 3

1) 200 g of ZSM-5 molecular sieve (silica-alumina molar ratio *n*(SiO₂): *n*(Al₂O₃) of 50, average particle size of 0.5 µm) and 500 ml of a 20 wt% silica sol (average particle size of 20 nm) were mixed uniformly;
2) 10 g of Cu(NO₃)₂ was added to 250 ml of a 20 wt% K₃PO₄ solution, added to the mixture of step 1), mixed uniformly, extruded and molded using an extruder, and calcined at 800°C for 2 h to obtain catalyst ZSM-1. The component contents measured by X-ray fluorescence method were: SiO₂: 94.41%, Al₂O₃: 1.24%, CuO: 2.05%, P₂O₅: 2.28%. The acid amount results of catalyst ZSM-1 are shown in Table 1.

The reaction was carried out using catalyst ZSM-1 according to the method and reaction conditions of Example 1. The reaction results are shown in Table 2.

### Example 4

The catalyst was prepared according to the method of Example 2, except that in step 2), 50 g of Cu₂SO₄ was added to 200 ml of a 25 wt% KH₂PO₄ solution, then the pH was adjusted to 4 with nitric acid, and then added to the mixture of step 1), and calcined at 500°C for 8 h to obtain catalyst β-2. The component contents measured by X-ray fluorescence method were: SiO₂: 74.09%, Al₂O₃: 19.47%, CuO: 2.88%, P₂O₅: 3.55%. The acid amount results of catalyst β-2 are shown in Table 1.

The reaction was carried out using catalyst β-2 according to the method and reaction conditions of Example 1. The reaction results are shown in Table 2.

### Example 5

The catalyst was prepared according to the method of Example 2, except that in step 2), 60 g of Cu₂SO₄ was added to 500 ml of a 10 wt% KH₂PO₄ solution, and the other conditions remained unchanged, to obtain catalyst β-3. The component contents measured by X-ray fluorescence method were: SiO₂: 73.68%, Al₂O₃: 19.36%, CuO: 3.44%, P₂O₅: 3.53%. The acid amount results of catalyst β-3 are shown in Table 1.

The reaction was carried out using catalyst β-3 according to the method and reaction conditions of Example 1. The reaction results are shown in Table 2.

### Example 6

The catalyst was prepared according to the method of Example 2, except that in step 2), 6 g of Cu₂SO₄ was added to 200 ml of a 25 wt% KH₂PO₄ solution, and the other conditions remained unchanged, to obtain catalyst β-4. The component contents measured by X-ray fluorescence method were: SiO₂: 76.03%, Al₂O₃: 19.98%, CuO: 0.36%, P₂O₅: 3.64%. The acid amount results of catalyst β-4 are shown in Table 1.

The reaction was carried out using catalyst β-4 according to the method and reaction conditions of Example 1. The reaction results are shown in Table 2.

### Example 7

The catalyst was prepared according to the method of Example 2, except that in step 1), alumina powder (average particle size of 20 µm) was used instead of the alumina sol, and the other conditions remained unchanged, to obtain catalyst β-5. The component contents measured by X-ray fluorescence method were substantially the same as in Example 2. The acid amount results of catalyst β-5 are shown in Table 1.

The reaction was carried out using catalyst β-5 according to the method and reaction conditions of Example 1. The reaction results are shown in Table 2.

### Comparative Example 1

The catalyst was prepared according to the method of Example 1, except that in step 2), a copper salt solution containing no phosphoric acid (100 ml of 3 wt% CuCl solution) was added, and the other conditions remained unchanged, to obtain catalyst DY-1. The component contents measured by X-ray fluorescence method were: SiO₂: 73.90%, Al₂O₃: 24.03%, CuO: 2.07%. The acid amount results of catalyst DY-1 are shown in Table 1.

The reaction was carried out using catalyst DY-1 according to the method and reaction conditions of Example 1. The reaction results are shown in Table 2.

### Comparative Example 2

The catalyst was prepared according to the method of Example 1, except that in step 2), a phosphoric acid solution containing no copper salt (100 ml of 10 wt% H₃PO₄ solution) was added, and the other conditions remained unchanged, to obtain catalyst DY-2. The component contents measured by X-ray fluorescence method were: SiO₂: 72.85%, Al₂O₃: 23.69%, P₂O₅: 3.46%. The acid amount results of catalyst DY-2 are shown in Table 1.

The reaction was carried out using catalyst DY-2 according to the method and reaction conditions of Example 1. The reaction results are shown in Table 2.

### Comparative Example 3

The catalyst was prepared according to the method of Example 2, except that in step 2), a copper salt solution containing no phosphate (100 ml of 5 wt% Cu₂SO₄ solution) was added, and the other conditions remained unchanged, to obtain catalyst Dβ-1. The component contents measured by X-ray fluorescence method were: SiO₂: 78.95%, Al₂O₃: 20.74%, CuO: 0.31%. The acid amount results of catalyst Dβ-1 are shown in Table 1.

The reaction was carried out using catalyst Dβ-1 according to the method and reaction conditions of Example 1. The reaction results are shown in Table 2.

### Comparative Example 4

The catalyst was prepared according to the method of Example 2, except that in step 2), a phosphate solution containing no copper salt (100 ml of 5 wt% KH₂PO₄ solution) was added, and the other conditions remained unchanged, to obtain catalyst Dβ-2. The component contents measured by X-ray fluorescence method were: SiO₂: 78.89%, Al₂O₃: 20.73%, P₂O₅: 0.38%. The acid amount results of catalyst Dβ-2 are shown in Table 1.

The reaction was carried out using catalyst Dβ-2 according to the method and reaction conditions of Example 1. The reaction results are shown in Table 2.

### Comparative Example 5

The catalyst was prepared according to the method of Example 2, except that in step 1), only β molecular sieve was added, and the other conditions remained unchanged, to obtain catalyst Dβ-3. The component contents measured by X-ray fluorescence method were: SiO₂: 94.46%, Al₂O₃: 4.72%, CuO: 0.37%, P₂O₅: 0.45%. The acid amount results of catalyst Dβ-3 are shown in Table 1.

The reaction was carried out using catalyst Dβ-3 according to the method and reaction conditions of Example 1. The reaction results are shown in Table 2.

### Comparative Example 6

The catalyst was prepared according to the method of Example 2, except that firstly, the molecular sieve was impregnated with the mixture formed by adding 5 g of Cu₂SO₄ to 100 ml of a 15 wt% KH₂PO₄ solution, then subjected to a first calcination (600°C, 1 h), then mixed with the alumina sol and molded, and then subjected to a second calcination (650°C, 4 h), to obtain catalyst Dβ-4. The component contents measured by X-ray fluorescence method were substantially the same as in Example 2. The acid amount results of catalyst Dβ-4 are shown in Table 1.

The reaction was carried out using catalyst Dβ-4 according to the method and reaction conditions of Example 1. The reaction results are shown in Table 2.

### Comparative Example 7

The catalyst was prepared according to the method of Example 2, except that in step 2), a zinc sulfate solution (100 ml of 15 wt% ZnSO₄ solution) was used instead of the phosphate solution, and the other conditions remained unchanged, to obtain catalyst Dβ-5. The component contents measured by X-ray fluorescence method were: SiO₂: 78.45%, Al₂O₃: 20.61%, CuO: 0.31%, ZnO: 0.63%. The acid amount results of catalyst Dβ-5 are shown in Table 1.

The reaction was carried out using catalyst Dβ-5 according to the method and reaction conditions of Example 1. The reaction results are shown in Table 2.

### Comparative Example 8

The catalyst was prepared according to the method of Example 2, except that in step 2), ferric chloride was used instead of copper sulfate, and the other conditions remained unchanged, to obtain catalyst Dβ-6. The component contents measured by X-ray fluorescence method were: SiO₂: 78.81%, Al₂O₃: 20.71%, Fe₂O₃: 0.11%, P₂O₅: 0.38%. The acid amount results of catalyst Dβ-6 are shown in Table 1.

The reaction was carried out using catalyst Dβ-6 according to the method and reaction conditions of Example 1. The reaction results are shown in Table 2.

### Comparative Example 9

The catalyst was prepared according to the method of Example 2, except that in step 2), nickel nitrate was used instead of copper sulfate, and the other conditions remained unchanged, to obtain catalyst Dβ-7. The component contents measured by X-ray fluorescence method were: SiO₂: 78.75%, Al₂O₃: 20.69%, NiO: 0.19%, P₂O₅: 0.38%. The acid amount results of catalyst Dβ-7 are shown in Table 1.

The reaction was carried out using catalyst Dβ-7 according to the method and reaction conditions of Example 1. The reaction results are shown in Table 2.

### Comparative Example 10

The catalyst was prepared according to the method of Example 2, except that in step 2), zinc sulfate was used instead of copper sulfate, and the other conditions remained unchanged, to obtain catalyst Dβ-8. The component contents measured by X-ray fluorescence method were: SiO₂: 78.73%, Al₂O₃: 20.69%, ZnO: 0.21%, P₂O₅: 0.38%. The acid amount results of catalyst Dβ-8 are shown in Table 1.

The reaction was carried out using catalyst Dβ-8 according to the method and reaction conditions of Example 1. The reaction results are shown in Table 2.

**Table 1**

| | | Weak acid amount /(µmol.g⁻¹) | Medium-strong acid amount /(µmol.g⁻¹) | Strong acid amount /(µmol.g⁻¹) | Total acid amount /(µmol.g⁻¹) | Medium-strong acid amount proportion/% |
|---|---|---|---|---|---|---|
| Ex. 1 | Y-1 | 907 | 821 | 153 | 1881 | 43.65 |
| Ex. 2 | β-1 | 703 | 654 | 107 | 1464 | 44.67 |
| Ex. 3 | ZSM-1 | 543 | 439 | 93 | 1075 | 40.84 |
| Ex. 4 | β-2 | 734 | 697 | 129 | 1560 | 44.68 |
| Ex. 5 | β-3 | 653 | 511 | 97 | 1261 | 40.52 |
| Ex. 6 | β-4 | 624 | 503 | 92 | 1219 | 41.26 |
| Ex. 7 | β-5 | 588 | 459 | 89 | 1136 | 40.40 |
| CE. 1 | DY-1 | 805 | 489 | 289 | 1583 | 30.89 |
| CE. 2 | DY-2 | 983 | 587 | 372 | 1942 | 30.23 |
| CE. 3 | Dβ-1 | 619 | 482 | 109 | 1210 | 39.83 |
| CE. 4 | Dβ-2 | 584 | 327 | 163 | 1074 | 30.45 |
| CE. 5 | Dβ-3 | 653 | 358 | 95 | 1106 | 32.37 |
| CE. 6 | Dβ-4 | 549 | 323 | 54 | 926 | 34.88 |
| CE. 7 | Dβ-5 | 501 | 248 | 88 | 837 | 29.63 |
| CE. 8 | Dβ-6 | 812 | 480 | 92 | 1384 | 34.68 |
| CE. 9 | Dβ-7 | 875 | 433 | 107 | 1415 | 30.60 |
| CE. 10 | Dβ-8 | 726 | 451 | 93 | 1270 | 35.51 |

From the results in Table 1 above, it can be seen that the catalysts according to the present disclosure, obtained by modifying both the molecular sieve and the oxide binder with P and Cu in combination, exhibited increased total acid amount and proportion of medium-strong acid amount, and effectively reduced the proportion of strong acid amount of the catalyst, compared with the catalysts of comparative examples obtained by modification with P alone or Cu alone, modification of molecular sieve alone with P and Cu in combination, modification of molecular sieve alone with P and Cu in combination followed by first calcination and then combination with oxide binder followed by second calcination, as well as modification with Zn and Cu in combination, modification with P and Fe in combination, modification with P and Ni in combination, and modification with P and Zn in combination.

**Table 2**

| | Catalyst | Aniline conversion rate/% | Lifetime/h | N-methyl aniline selectivity/ % | N,N-dimethyl aniline selectivity/ % | N,N,C-trimethyl aniline selectivity/ % |
|---|---|---|---|---|---|---|
| Ex. 1 | Y-1 | 100 | 400 | 5.14 | 94.85 | 0.01 |
| Ex. 2 | β-1 | 99.9 | 800 | 4.03 | 95.96 | 0.01 |
| Ex. 3 | ZSM-1 | 99.5 | 300 | 6.12 | 93.86 | 0.02 |
| Ex. 4 | β-2 | 99.9 | 780 | 3.95 | 96.02 | 0.03 |
| Ex. 5 | β-3 | 99.8 | 750 | 3.85 | 96.13 | 0.02 |
| Ex. 6 | β-4 | 99.8 | 660 | 4.81 | 95.175 | 0.015 |
| Ex. 7 | β-5 | 99.5 | 620 | 5.01 | 94.96 | 0.03 |
| CE. 1 | DY-1 | 100 | 80 | 6.55 | 92.89 | 0.56 |
| CE. 2 | DY-2 | 100 | 260 | 7.14 | 92.15 | 0.71 |
| CE. 3 | Dβ-1 | 99.1 | 335 | 6.87 | 92.54 | 0.59 |
| CE. 4 | Dβ-2 | 95.2 | 402 | 8.66 | 90.71 | 0.63 |
| CE. 5 | Dβ-3 | 98.3 | 212 | 40.25 | 59.23 | 0.52 |
| CE. 6 | Dβ-4 | 97.2 | 586 | 5.42 | 93.35 | 1.23 |
| CE. 7 | Dβ-5 | 98.7 | 107 | 10.47 | 88.74 | 0.79 |
| CE. 8 | Dβ-6 | 97.4 | 680 | 3.45 | 92.83 | 3.72 |
| CE. 9 | Dβ-7 | 99.2 | 590 | 7.22 | 90.77 | 2.01 |
| CE. 10 | Dβ-8 | 99.1 | 746 | 5.07 | 93.92 | 1.01 |

From the results in Table 2 above, it can be seen that the catalysts according to the present disclosure, obtained by modifying both the molecular sieve and the oxide binder with P and Cu in combination, effectively reduced the selectivity of the main by-products N-methylaniline and N,N,C-trimethylaniline, and significantly improved the lifetime of the catalyst.

### Example 8

The catalyst was prepared according to the method of Example 2, except that after calcination, the temperature was lowered to 350°C, and hydrogen was introduced (hydrogen flow rate of 10 mL/min/g catalyst) for activation for 2 h to obtain catalyst β-6. The component contents measured by X-ray fluorescence spectrometry were substantially the same as in Example 2. The Cu⁺/Cu²⁺ ratio and pyridine infrared acid amount results of catalyst β-6 are shown in Table 3.

The reaction was carried out using catalyst β-6 according to the method and reaction conditions of Example 1. The reaction results are shown in Table 4.

### Example 9

The catalyst was prepared according to the method of Example 8, except that in step 2), 100 g of Cu₂SO₄ and 12 g of zinc sulfate were added to 100 ml of a 20 wt% KH₂PO₄ solution, and the other conditions remained unchanged, to obtain catalyst β-7. The component contents measured by X-ray fluorescence spectrometry were: SiO₂: 73.98%, Al₂O₃: 19.44%, CuO: 5.76%, P₂O₅: 0.35%, ZnO: 0.48%. The Cu⁺/Cu²⁺ ratio and pyridine infrared acid amount results of catalyst β-7 are shown in Table 3.

The reaction was carried out using catalyst β-7 according to the method and reaction conditions of Example 1. The reaction results are shown in Table 4.

### Example 10

The catalyst was prepared according to the method of Example 8, except that in step 2), 10 g of Cu₂SO₄ and 1.5 g of nickel nitrate were added to 100 ml of a 10 wt% K₃PO₄ solution, and the other conditions remained unchanged, to obtain catalyst β-8. The component contents measured by X-ray fluorescence spectrometry were: SiO₂: 78.54%, Al₂O₃: 20.46%, CuO: 0.61%, P₂O₅: 0.16%, NiO: 0.06%. The Cu⁺/Cu²⁺ ratio and pyridine infrared acid amount results of catalyst β-8 are shown in Table 3.

The reaction was carried out using catalyst β-8 according to the method and reaction conditions of Example 1. The reaction results are shown in Table 4.

### Example 11

The catalyst was prepared according to the method of Example 8, except that in step 2), 100 g of Cu₂SO₄ and 12 g of ferrous chloride were added to 100 ml of a 10 wt% KH₂PO₄ solution, and the other conditions remained unchanged, to obtain catalyst β-9. The component contents measured by X-ray fluorescence spectrometry were: SiO₂: 74.10%, Al₂O₃: 19.47%, CuO: 5.77%, P₂O₅: 0.35%, Fe₂O₃: 0.30%. The Cu⁺/Cu²⁺ ratio and pyridine infrared acid amount results of catalyst β-9 are shown in Table 3.

The reaction was carried out using catalyst β-9 according to the method and reaction conditions of Example 1. The reaction results are shown in Table 4.

**Table 3**

| | Catalyst | Cu⁺/Cu²⁺ | L acid amount /(µmol.g⁻¹) | B acid amount /(µmol.g⁻¹) | Pyridine infrared total acid amount /(µmol.g⁻¹) | Proportion of L acid amount in pyridine infrared total acid amount (%) |
|---|---|---|---|---|---|---|
| Ex. 2 | β-1 | 0 | 472 | 247 | 719 | 65.65% |
| Ex. 4 | β-2 | 0 | 596 | 203 | 799 | 74.59% |
| Ex. 5 | β-3 | 0 | 585 | 149 | 734 | 79.70% |
| Ex. 6 | β-4 | 0 | 492 | 247 | 739 | 66.58% |
| Ex. 7 | β-5 | 0 | 488 | 233 | 721 | 67.68% |
| Ex. 8 | β-6 | 0.15 | 451 | 225 | 676 | 66.72% |
| Ex. 9 | β-7 | 0.23 | 501 | 233 | 734 | 68.26% |
| Ex. 10 | β-8 | 0.29 | 564 | 163 | 727 | 77.58% |
| Ex. 11 | β-9 | 0.35 | 528 | 246 | 774 | 68.22% |

**Table 4**

| | Catalyst | Aniline conversion rate/% | Lifetime/h | N-methyl aniline selectivity/ % | N,N-dimethyl aniline selectivity/ | N,N,C-trimethyl aniline selectivity/ |
|---|---|---|---|---|---|---|
| | | | | | % | % |
| Ex. 2 | β-1 | 99.9 | 800 | 4.03 | 95.96 | 0.01 |
| Ex. 8 | β-6 | 100 | 2200 | 3.95 | 96.04 | 0.01 |
| Ex. 9 | β-7 | 99.9 | 2580 | 3.79 | 96.20 | 0.01 |
| Ex. 10 | β-8 | 100 | 2385 | 3.70 | 96.29 | 0.01 |
| Ex. 11 | β-9 | 100 | 2240 | 4.88 | 95.11 | 0.01 |

From the results in Tables 3 and 4 above, it can be seen that the catalysts according to the present disclosure, by adjusting the ratio of Cu⁺/Cu²⁺, further greatly improved the activity and stability of the catalyst, particularly significantly improving the lifetime of the catalyst.

### Example 12

The reaction was carried out using catalyst β-1 according to the method and reaction conditions of Example 1, except that the reaction temperature was 230°C. The reaction results are shown in Table 5.

### Example 13

The reaction was carried out using catalyst β-6 according to the method and reaction conditions of Example 1, except that the reaction temperature was 230°C. The reaction results are shown in Table 5.

### Example 14

The reaction was carried out using catalyst β-7 according to the method and reaction conditions of Example 1, except that the reaction temperature was 230°C. The reaction results are shown in Table 5.

### Example 15

The reaction was carried out using catalyst β-8 according to the method and reaction conditions of Example 1, except that the reaction temperature was 230°C. The reaction results are shown in Table 5.

### Example 16

The reaction was carried out using catalyst β-9 according to the method and reaction conditions of Example 1, except that the reaction temperature was 230°C. The reaction results are shown in Table 5.

### Comparative Example 11

The reaction was carried out using catalyst Dβ-1 according to the method and reaction conditions of Example 1, except that the reaction temperature was 230°C. The reaction results are shown in Table 5.

### Comparative Example 12

The reaction was carried out using catalyst Dβ-2 according to the method and reaction conditions of Example 1, except that the reaction temperature was 230°C. The reaction results are shown in Table 5.

**Table 5**

| | Catalyst | Aniline conversion rate/% | Lifetime/h | N-methyl aniline selectivity /% | N,N-dimethyl aniline selectivity /% | N,N,C-trimethyl aniline selectivity /% |
|---|---|---|---|---|---|---|
| Ex. 12 | β-1 | 99.9 | 900 | 1.80 | 98.19 | 0.01 |
| Ex. 13 | β-6 | 100 | 2650 | 2.01 | 97.97 | 0.02 |
| Ex. 14 | β-7 | 100 | 2800 | 2.12 | 97.87 | 0.01 |
| Ex. 15 | β-8 | 100 | 2740 | 2.33 | 97.66 | 0.01 |
| Ex. 16 | β-9 | 99.9 | 2680 | 1.96 | 98.02 | 0.02 |
| CE. 11 | Dβ-1 | 53.3 | - | 2.7 | 97.3 | 0 |
| CE. 12 | Dβ-2 | 62.4 | - | 1.9 | 98.1 | 0 |

From the results in Table 5 above, it can be seen that when using the catalyst of the present disclosure in the synthesis of N,N-dimethylaniline at a low temperature (230°C), the conversion rate of the raw material aniline still reached more than 99%, the catalyst lifetime was longer than that at a high temperature (250°C), the catalyst was more stable, and side reactions were suppressed at the low temperature (230°C), resulting in higher N,N-dimethylaniline selectivity. The catalyst provided by the present disclosure is suitable for the synthesis of N,N-dimethylaniline both at high temperatures and at low temperatures, thereby broadening the applicable reaction temperature range of the N-alkylation reaction, which is beneficial for realizing long-period operation of the N,N-dimethylaniline production process.

### Example 17

The reaction was carried out using catalyst β-1 according to the method and reaction conditions of Example 1, and after the conversion rate of aniline decreased to 98% (1st cycle), the used catalyst was recovered by gas-solid separation and regenerated, and then put into the next cycle. The reaction was cycled 4 times. The evaluation results of recycling performance are shown in Table 6.

**Table 6. Recycling performance of catalyst β-1**

| Number of cycles | Lifetime /h | N-methyl aniline selectivity/% | N,N-dimethyl aniline selectivity/% | N,N,C-trimethyl aniline selectivity/% |
|---|---|---|---|---|
| 1st | 800 | 4.03 | 95.96 | 0.01 |
| 2nd | 790 | 3.97 | 96.02 | 0.01 |
| 3rd | 810 | 4.52 | 95.46 | 0.02 |
| 4th | 795 | 4.11 | 95.88 | 0.01 |

From the results in Table 6 above, it can be seen that the catalyst according to the present disclosure showed no significant decrease in catalytic performance and service life after 4 cycles, had good recycling performance, and could be reused for the continuous production of N,N-dimethylaniline, which is beneficial for saving production costs.

The specific embodiments of the present disclosure have been described in detail above, but the present disclosure is not limited thereto. Within the technical concept of the present disclosure, various simple modifications can be made to the technical solutions of the present disclosure, including any other suitable combinations of the various technical features. These simple modifications and combinations should also be regarded as the content disclosed by the present disclosure and all fall within the protection scope of the present disclosure.

## Claims

1. An N-alkylation catalyst, **characterized in that**, the catalyst comprises a molecular sieve, an oxide binder, and a modifying component, the modifying component is supported on both the molecular sieve and the oxide binder, the modifying component comprises Cu and P, the percentage of the medium-strong acid amount with respect to the total acid amount of the catalyst is not less than 35%, preferably not less than 40%, more preferably not less than 42%, the percentage of the medium-strong acid amount in the total acid amount is calculated as the percentage of the desorption peak area in a range of 250-450°C with respect to the desorption peak area of the total acid amount measured by NH₃ temperature-programmed desorption method.

2. The catalyst according to claim 1, **characterized in that**, the molecular sieve is selected from at least one of Y-type molecular sieve, ZSM-5 type molecular sieve, β-type molecular sieve, MCM-22 type molecular sieve, and mordenite type molecular sieve, preferably at least one of Y-type molecular sieve, ZSM-5 type molecular sieve, and β-type molecular sieve, more preferably β-type molecular sieve, and/or
the oxide binder is alumina and/or silica.

3. The catalyst according to any one of preceding claims, **characterized in that**,
the Cu:P molar ratio calculated as oxides *n*(CuO): *n*(P₂O₅) in the catalyst is in a range of 30:1-1:25, preferably in a range of 20:1-1:10; and/or
the mass ratio of the molecular sieve to the oxide binder is in a range of 95:5-20:80, preferably in a range of 90:10-25:75.

4. The catalyst according to any one of preceding claims, **characterized in that**, the ratio of Cu⁺/Cu²⁺ in the catalyst is in a range of 0-0.4, preferably in a range of 0.1-0.35; and/or,
the percentage of the pyridine infrared L acid amount with respect to the pyridine infrared total acid amount of the catalyst is not less than 60%, preferably in a range of 65-80%.

5. The catalyst according to any one of preceding claims, **characterized in that**, the modifying component further comprises a metal M component selected from at least one of Group VIII metals and Group VIB metals; preferably, the metal M component is selected from at least one of Cr, Zn, Fe, and Ni, more preferably selected from at least one of Cr, Zn, and Ni.

6. A method for preparing an N-alkylation catalyst, **characterized in that**, the method comprises:
1) mixing a molecular sieve and an oxide binder to obtain a mixture;
2) mixing the mixture obtained in step 1) with a solution containing a copper precursor and a phosphorus-containing compound, followed by molding, and then subjecting to a single calcination, to obtain a catalyst.

7. The preparation method according to claim 6, wherein, in step 1), the oxide binder is a solid-state oxide binder or a sol-state oxide binder, preferably a sol-state oxide binder; preferably the concentration of the sol-state oxide binder is in a range of 0.1-50 wt%, more preferably in a range of 10-30 wt%; preferably the pH of the sol-state oxide binder is in a range of 1-5; preferably the average particle size of the sol-state oxide binder is in a range of 0.1-300 nm; and/or,
in step 1), the oxide binder is alumina and/or silica, more preferably an alumina sol and/or a silica sol; and/or,
in step 1), the molecular sieve is selected from at least one of Y-type molecular sieve, ZSM-5 type molecular sieve, β-type molecular sieve, MCM-22 type molecular sieve, and mordenite, preferably at least one of Y-type molecular sieve, ZSM-5 type molecular sieve, and β-type molecular sieve, more preferably β-type molecular sieve; preferably, the silica-alumina molar ratio *n*(SiO₂): *n*(Al₂O₃) of the molecular sieve is in a range of 2-1000:1, preferably in a range of 2-100:1; preferably, the average particle size of the molecular sieve is in a range of 0.01-50 µm, preferably in a range of 0.1-20 µm.

8. The preparation method according to any one of claims 6-7, wherein,
in step 2), the copper precursor is selected from soluble copper compounds, preferably selected from at least one of copper chloride, cuprous chloride, copper nitrate, copper sulfate, and cuprous sulfate; and/or,
in step 2), the phosphorus-containing compound is selected from at least one of phosphoric acid, phosphorous acid, soluble phosphates and phosphites; preferably, the soluble phosphate is selected from at least one of ammonium phosphate, diammonium hydrogen phosphate, and ammonium dihydrogen phosphate.

9. The preparation method according to any one of claims 6-8, wherein, in step 2), calculated as elements, the mass ratio of the copper precursor to the phosphorus-containing compound is in a range of 5:95-98:2, preferably in a range of 30:70-80:20; and/or,
the mass ratio of the oxide binder to the copper precursor calculated as elements is in a range of 80:20-99.9:0.1, preferably in a range of 85:15-99:1.

10. The preparation method according to any one of claims 6-9, wherein, in step (2), the calcination is conducted under conditions including: temperature in a range of 450-800°C, preferably in a range of 500-700°C; time in a range of 1-15 h, preferably in a range of 1-12 h.

11. The preparation method according to any one of claims 6-10, wherein, when a sol-state oxide binder is used in step 1), the method further comprises: introducing a peptizing agent into the solution containing the copper precursor and the phosphorus-containing compound, and mixing with the mixture obtained in step 1), followed by molding; preferably, the peptizing agent is selected from at least one of nitric acid, hydrochloric acid, sulfuric acid, phosphoric acid, formic acid, and acetic acid; preferably, the peptizing agent is used in such an amount that the pH of the solution containing the copper precursor and the phosphorus-containing compound is in a range of 1-4.

12. The preparation method according to any one of claims 6-11, wherein, the method further comprises: 3) subjecting the catalyst obtained in step 2) to an activation treatment to obtain an activated catalyst;
preferably, the activation treatment is conducted under an activation atmosphere, preferably the activation atmosphere is selected from at least one of hydrogen, CO, ammonia, and hydrogen sulfide, more preferably hydrogen;
preferably, the activation treatment is conducted under conditions including: temperature in a range of 150-600°C, preferably in a range of 200-400°C; time in a range of 0.5-30 h, preferably in a range of 2-24 h; flow rate of the activation atmosphere in a range of 0.001-100 mL/min/g catalyst, preferably in a range of 0.01-50 mL/min/g catalyst.

13. The preparation method according to any one of claims 6-12, wherein, step 2) further comprises: introducing a metal M component precursor to mix with the mixture obtained in step 1), the copper precursor, and the phosphorus-containing compound,
preferably, calculated as elements, the mass ratio of the metal M component precursor to the copper precursor is in a range of 1:50-1:1, preferably in a range of 1:25-1:2;
preferably, the metal M component precursor is selected from soluble compounds of at least one of Group VIII metals and Group VIB metals, preferably selected from soluble compounds of at least one of Cr, Zn, Fe, and Ni, more preferably selected from at least one of chromium chloride, chromium nitrate, zinc nitrate, zinc sulfate, nickel nitrate, basic nickel carbonate, nickel acetate, nickel sulfate, iron nitrate, ferrous chloride, and ferric chloride.

14. A method for producing N,N-dimethylaniline, **characterized in that**, the method comprises subjecting methanol and aniline to a reaction in the presence of a catalyst to produce N,N-dimethylaniline, wherein the reaction is a gas phase reaction; wherein, the catalyst is the catalyst according to any one of claims 1-5 or the catalyst prepared by the method according to any one of claims 6-13.

15. The method according to claim 14, wherein, the reaction is conducted under conditions including: molar ratio of methanol to aniline in a range of 1-10:1, preferably in a range of 2-8:1; reaction temperature in a range of 180-350°C, preferably in a range of 220-300°C; weight hourly space velocity of methanol and aniline in a range of 0.1-10 h⁻¹, preferably in a range of 0.5-4.5 h⁻¹.
